(19) **Europäisches Patentamt / European Patent Office / Office européen des brevets**

(11) **EP 2 299 898 B1**

(12) # EUROPEAN PATENT SPECIFICATION

(45) Date of publication and mention
of the grant of the patent:
**05.02.2020 Bulletin 2020/06**

(21) Application number: **09787992.8**

(22) Date of filing: **10.07.2009**

(51) Int Cl.:
**A61B 5/00** (2006.01)

(86) International application number:
**PCT/JP2009/062930**

(87) International publication number:
**WO 2010/005116 (14.01.2010 Gazette 2010/02)**

(54) **BIOLOGICAL INFORMATION ACQUISITION APPARATUS**

VORRICHTUNG ZUR BESCHAFFUNG BIOLOGISCHER INFORMATIONEN

DISPOSITIF D ACQUISITION D INFORMATIONS BIOLOGIQUES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **11.07.2008 JP 2008182055**
**06.01.2009 JP 2009000891**

(43) Date of publication of application:
**30.03.2011 Bulletin 2011/13**

(73) Proprietor: **Canon Kabushiki Kaisha**
**Tokyo 146-8501 (JP)**

(72) Inventors:
- **ICHIHARA, Shigeru**
 **Tokyo 146-8501 (JP)**
- **KOBAYASHI, Shuichi**
 **Tokyo 146-8501 (JP)**

(74) Representative: **TBK**
**Bavariaring 4-6**
**80336 München (DE)**

(56) References cited:
**WO-A-2005/068973      US-A- 5 348 002**
**US-A1- 2005 090 725      US-A1- 2006 184 042**

**Description**

TECHNICAL FIELD

The present invention relates to a biological information acquisition apparatus, more particularly to an apparatus for acquiring biological information on the basis of a photoacoustic effect and imaging the acquired biological information.

BACKGROUND ART

In recent years, photoacoustic tomography (PAT), using the characteristic of an ultrasonic wave to scatter less in a living body than that of light, and acquiring an optical characteristic value distribution in the living body at a high resolution, has been proposed (see M. Xu, L. V. Wahng "Photoacoustic imaging biomedicine, Review of scientific instruments, 77, 04111 (2006)).

When a pulse light is generated from a light source and radiated to a living body, the pulse light diffuses in the living body and propagates therein.

Then an absorber included in a living body tissue absorbs the energy of the propagated pulse light and generates an acoustic wave.

The acoustic wave is received by an acoustic wave detector and is converted into an electric signal by the acoustic wave detector. The converted electric signal is subjected to analysis processing, and thereby the optical characteristic distribution, especially the optical energy absorption density, of the living body can be acquired.

According to Xu et al., in the PAT, the sound pressure (P) of the sound wave acquired from a light absorption by an absorber in a living body can be expressed by the following formula.

$$P = \Gamma \cdot \mu_a \cdot \Phi$$

The character $\Gamma$ denotes a Gruneisen coefficient, which is an elastic characteristic value and is acquired by dividing a product of a coefficient of volume expansion ($\beta$) and the square of the acoustic velocity (c) by specific heat ($C_p$) here. The character $\mu_a$ denotes the coefficient of absorption of the absorber, and the character $\Phi$ denotes a light quantity in a local region (the quantity of light radiated to the absorber).

The sound pressure, which is used as a signal of an acoustic wave by the PAT, is proportioned to the local quantity of the light that reaches the absorber. Because the light irradiated onto a living body is rapidly attenuated in the body by being scattered and absorbed, the sound pressure of an acoustic wave generated in a deep tissue in the body is greatly attenuated according to the distance from a light irradiation part. As the detection methods of acoustic waves, a back detection method of making a light enter a detector from the same direction as that of the detector to detect an acoustic wave, and a forward detection method of making a light enter a detector from the opposed direction to the detector to detect an acoustic wave, are known.

In the back detection PAT, PAT microscopy of making a light obliquely enter an object from a side (side surface side) of an acoustic wave detector in order to radiate a light effectively to the back of the acoustic wave detector, and the like have been proposed (see J. T. Niederhauser, M. Jeager, R. Lemor, P. Weber, and M. Frenz, IEEE Transactions on medical imaging, vol. 24, No. 4, 436 (2005), Japanese Patent Application Laid-Open No. 2008-049063, United State Patent Application Publication No. 2006/0184042).

J. T. Niederhauser et al. and U.S. Patent Publication Application No. 2006/0184042 chiefly aim at illuminating a specific part in a living body with a light by arranging mirrors and lenses on a side of an acoustic wave detector.

On the other hand, Japanese Patent Application Laid-Open No. 2008-049063 illuminates a living body tissue with a light by means of a structure of providing a light source on a side (side surface side) of an acoustic wave detector directly.

The US 2006/184042 A1 discloses a laser photoacoustic imaging system capable of producing a three-dimensional image (tomographic scan) of human organs. A photoacoustic sensor of the imaging system comprises an ultrasonic transducer attached to a concave lens. Laser light from optical fibers is focused through an optical condenser. The optical focal region overlaps with the focal spot of the ultrasonic transducer, thus forming a confocal optical dark-field illumination and ultrasonic detection configuration.

The WO 2005/068973 A discloses a photoacoustic sensor. Light that enters a light pipe from an optic fiber is coupled to an output aperture so that it exits the aperture using e.g. microprisms. The light pipe may comprise microprisms to couple light from the optic fiber to the output aperture. The microprisms are optionally formed on a region of a top surface of the light pipe opposite the optical aperture. The microprisms reflect and refract a portion of light incident on the microprisms towards the optical aperture at angles that are greater than the critical angle for the light, so that the light, when it is incident on the optical aperture, exits the light pipe.

Further relevant prior art is found in the US 2005/090725 A1 and the US 5 348 002 A.

DISCLOSUSRE OF THE INVENTION

In a diagnosis of a living body, condensing light onto a specific part as disclosed in U.S. Patent Publication Application No. 2006/0184042 can be an appropriate technique as far as a chief object is to perform a detailed observation to a known disease part.

However, in case of performing a screening diagnosis of an unknown disease like a diagnosis of breast cancer, it is required to radiate a more uniform light to the whole desired living body tissue.

The present invention is directed to provide a biological information acquisition apparatus capable of radiating a uniform and efficient light to the whole living body tissue as an object of the detection.

The present invention provides an information acquisition apparatus configured as defined in the appended claims.

According to the present invention, a biological information acquisition apparatus capable of uniform and efficient illumination to an object including a living body tissue can be realized.

Further features of the present invention will become apparent from the following description of exemplary embodiments with reference to the attached drawings.

BRIEF DESCRIPTION OF THE DRAWINGS

FIG. 1 is a schematic view of back detection PAT arranging an acoustic wave detector in the direction same as the incident direction of a light;

FIG. 2 is a schematic view of forward detection PAT arranging an acoustic wave detector in a direction opposed to the incident direction of a light;

FIG. 3 is a schematic view of both-side irradiation PAT making lights enter an object from both surfaces and arranging a detector on one surface;

FIG. 4 is a schematic view illustrating a configuration example of a back detection PAT in a first embodiment of the present invention;

FIG. 5 is a sectional view of an acquisition range and illumination region of an acoustic wave signal of the back detection PAT in the first embodiment of the present invention;

FIG. 6 is a schematic view illustrating the illumination region of the back detection PAT in the first embodiment of the present invention;

FIG. 7 is a schematic view illustrating a back detection PAT of a comparative example;

FIG. 8 is a schematic view illustrating a form illustrating an optical path;

FIG. 9 is a schematic view illustrating a configuration example of back detection PAT in a second embodiment of the present invention;

FIG. 10 is a schematic view illustrating an illumination region of the back detection PAT in the second embodiment of the present invention;

FIG. 11 is a schematic view illustrating a configuration example of both-side irradiation PAT in a third embodiment of the present invention;

FIG. 12 is a view illustrating an example of PAT to which the present invention can be applied;

FIG. 13 is a schematic view illustrating an embodiment of back detection PAT to which the present invention can be applied; and

FIG. 14 is a schematic view illustrating an embodiment of back detection PAT to which the present invention can be applied.

BEST MODE FOR CARRYING OUT THE INVENTION

Next, photoacoustic tomography (hereinafter referred to as PAT) in embodiments to which the present invention is applied will be described. The same constituent elements will be denoted by the same reference numerals in principle.

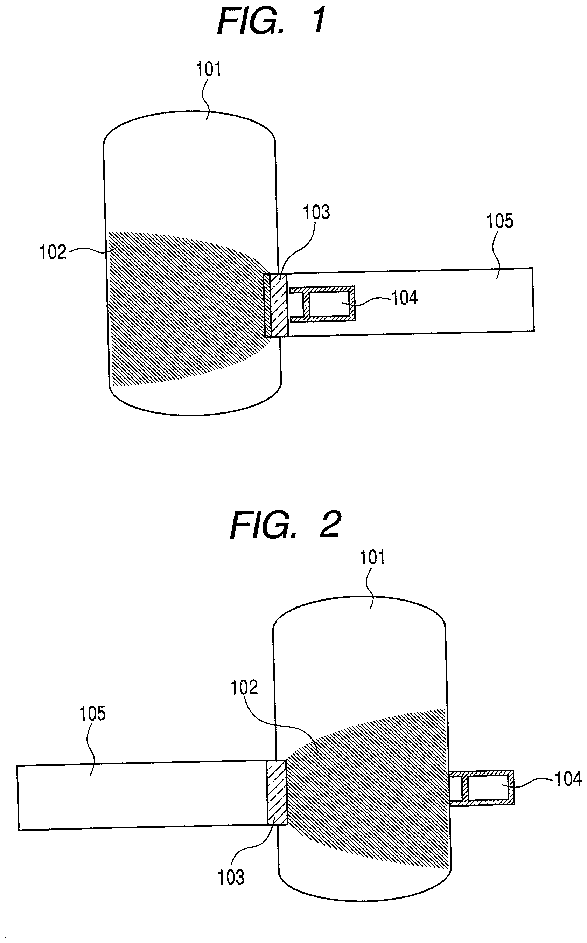

As illustrated in FIG. 1, the case of arranging an acoustic wave detector 104 on the side same as the incident side (also referred to as incidence plane side) of a light into an object is defined as back detection PAT here.

As illustrated in FIG. 2, the case of arranging the acoustic wave detector 104 on the side opposed to the incident side of a light into the object is defined as forward detection PAT.

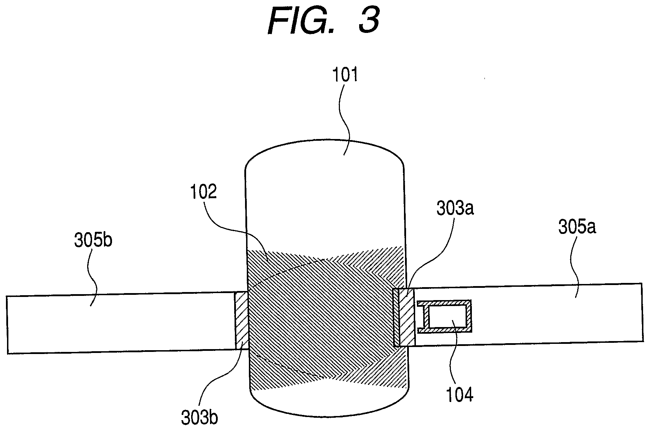

As illustrated in FIG. 3, the case of forming two illuminating areas 303a and 303b and two illuminating light paths 305a and 305b by making lights enter the object from both opposed surfaces with the object put between them and of arranging the detector 104 on one of the opposed surfaces is defined as both-side irradiation PAT.

The biological information acquisition apparatus of the present embodiment has the following configuration in order to relax the restriction of a critical angle and to increase an incident light quantity.

The biological information acquisition apparatus of the present embodiment includes a first pulse light source and the acoustic wave detector detecting an acoustic wave generated by making a light from the pulse light source enter the object through an object holding member.

Moreover, one or more light transmitting members are provided to be arranged, and a light from the light source enters the light transmitting members from a side surface of the acoustic wave detector and enters the object holding member at an angle other than one perpendicular to the object holding member through the light transmitting members.

The light transmitting member in the present invention indicates a member absorbing a small quantity of the light in near-infrared/visible regions to be used and having a transmittance of 90% or more of the light. It is preferable that the light transmitting member has a transmittance approximate to 100%.

The object holding member indicates a member provided between the light source to hold the object, and the object holding member includes the members called as a flat surface plate, a pressure paddle, a parallel plate, and a plate.

An acoustic wave in the present invention indicates an elastic wave generated within an object when a light, such as a near-infrared ray, is radiated to the object, and includes the waves called as a sound wave, an ultrasonic wave, and a photoacoustic wave.

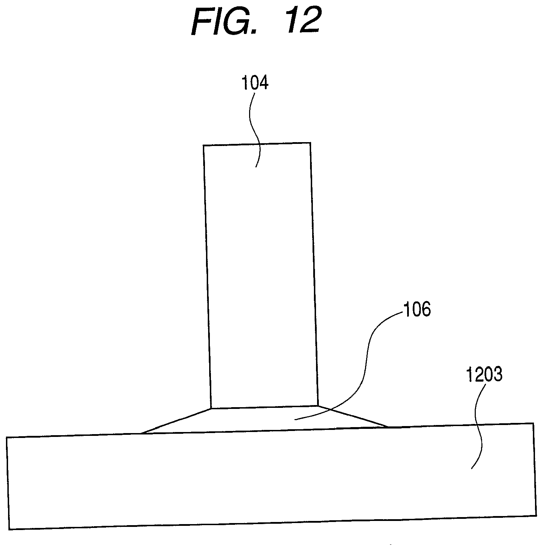

As illustrated in FIG. 12, the light transmitting member 106 may be arranged between an acoustic wave detector 104 and an object holding member 1203. However, if the thickness of the light transmitting member 106 is too large, a signal from the object sometimes attenuates so much before the signal reaches the detector 104.

Accordingly if the light transmitting member 106 is arranged between the acoustic wave detect 104 and the object holding member 1203, then it is preferable to design the thickness of the light transmitting member 106 to be within a range in which necessary sensitivity can be acquired.

The light transmitting member 106 can be configured to be integrated with the acoustic wave detector 104 as a unit structure.

Because the unit structure can extensively detect acoustic waves by performing two-dimensional scanning over the object holding member 1203, the two-dimensional scanning is preferable.

When the refractive index of the light transmitting member 106 is denoted by a character n1 and the refractive index of the object holding member 1203 is denoted by a character n2, it is preferable to configure the light transmitting member 106 and the object holding member 1203 so as to satisfy the relation of n1 > n2. By adjusting the magnitudes of the refractive indices of the light transmitting member 106 and the object holding member 1203 as described above, it is possible to make a light enter the object efficiently.

The light transmitting member 106 and the object holding member 1203 may be configured so that the refractive indices satisfy the relation of n1 < n2.

In the biological information acquisition apparatus of the present embodiment, the configuration of providing the light transmitting member 106 to be arranged on both the sides of the acoustic wave detector 104 (side surfaces surrounding the detection surface of the acoustic wave detector 104) can be adopted. Moreover, in the biological information acquisition apparatus of the present embodiment, the configuration in which a lubricant is supplied (or filled up) between the object holding member 1203 and the unit structure can be adopted. Moreover, in the biological information acquisition apparatus of the present embodiment, the configuration of satisfying the relation of n1 > n3 > n2 can be adopted, where the characters n1, n2, and n3 denote the refractive indices of the light transmitting member 106, the object holding member 1203, and the lubricant, respectively.

By satisfying this relation of the refractive indices n1, n2, and n3, the reflectance occurring on the interface of each of the light transmitting member 106, the object holding member 1203, and the lubricant can be reduced, and a light can be made to enter the object efficiently.

In the biological information acquisition apparatus of the present embodiment, the acoustic wave detector 104 can be configured of a 2-dimensional array acoustic wave detecting device.

Moreover, the biological information acquisition apparatus of the present embodiment can be configured to include a parallel flat plate member arranged to be opposed to and in parallel with the object holding member 1203 with the object put between them, a pressure mechanism pressing the object with the two flat plates of the object holding member 1203 and the parallel flat plate member, and a second light source, different from the first light source, radiating a light from the parallel flat plate member side to the object.

Moreover, the biological information acquisition apparatus of the present embodiment can be configured to use the second pulse light source in integrated with the first pulse light source.

(First Embodiment)

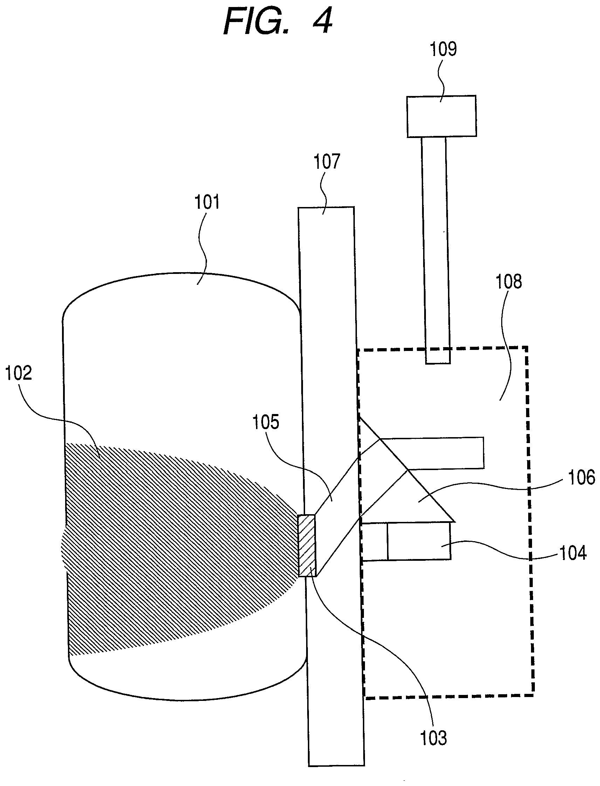

Next, as a first embodiment, a configuration example of the back detection PAT will be described. FIG. 4 illustrates a view for describing the configuration example of the back detection PAT of the first embodiment.

In the back detection PAT of the present embodiment, a diffusion light area 102 and an illuminating area 103 are formed in a living body tissue 101 as an object of the detection. The apparatus of the back detection PAT includes a flat surface plate 107 as the object holding member, the acoustic wave detector 104, the light transmitting member 106, and a light source.

An illuminating light path 105 is a path of the light radiated from the light source to the present apparatus.

The acoustic wave detector 104 and the light transmitting member 106 are integrated to form a unit structure 108 here. The present apparatus is configured so that the unit structure 108 two-dimensionally scans the flat surface plate 107 to detect the acoustic wave generated from an absorber in the living body by means of a unit position control unit 109 and by through the lubricant which is also used also as an acoustic wave matching agent.

The acquired acoustic wave is converted into an electric signal, and the imaging of the biological information is performed by the signal processing and image construction algorithm using the electric signal.

Incidentally, any signal processing unit may be used as long as the signal processing unit can store the strength of an acoustic wave and the time changes of the strength and can convert the stored strength and time changes into the data of an optical characteristic value distribution by an operation unit.

For example, an oscilloscope and a computer capable of analyzing the data stored in the oscilloscope can be used as the signal processing unit.

The same kind of the acoustic wave detector of a conventionally widely used acoustic wave diagnosis apparatus is suitably used as the acoustic wave detector 104.

The acoustic wave detector 104 detects an acoustic wave (elastic wave) generated by a light absorber in a living body that has absorbed a part of energy of a light radiated to the living body, and converts the detected acoustic wave into an electric signal.

As the acoustic wave detector 104, any acoustic wave detector, such as the one using piezoelectric phenomenon, the one using the resonance of a light, and the one using the changes of capacity, can be used as long as the acoustic wave detector can detect an acoustic wave signal.

It is preferable that the acoustic wave detector 104 is composed of a 2-dimensional array acoustic wave detecting device, and it is possible to design the acoustic wave detector 104 as the one having the number of elements according to a signal processing circuit.

Although the description will be given by using acoustic wave detection elements arranged lengthwise and breadthwise of a two-dimensional arrangement in the present embodiment, the shapes of elements are not limited to this type. As well, the pitch between the elements may also be set arbitrarily.

As the light source, a light source generating a pulse light can be used.

The pulse light from the pulse light source is the one having the pulse width of the order of several nanoseconds to several hundreds nanoseconds, and preferably has the wavelength within a range from 400 nm to 1600 nm, both inclusive.

In particular, it is preferable to use a solid state laser, such as an Nd: YAG laser and an Alexandrite laser, having a strong irradiation energy intensity.

It is enabled to measure the differences of the optical characteristic value distributions owing to wavelengths by the use of a dye laser, optical parametric oscillators (OPO) laser, or a Ti: sapphire laser, which can convert an oscillating wavelength.

The region from 700 nm to 1100 nm, in which the absorption of light is little in a living body, is more preferable as to the wavelength of the light source to be used.

In the back detection PAT, because the acoustic wave detector 104 and the illumination optics are arranged on the same side and an optically shielded region is easily produced, it is necessary to consider how to illuminate the object.

In particular, as the detection area of the acoustic wave detector 104 increases, the problem is actualized. Although the technique of embedding an optical waveguide, such as an optical fiber, in the detection surface of the acoustic wave detector 104 to perform light illumination was proposed, the use of the optical fiber is limited to the use under its thermotolerance temperatures.

The relation between the acoustic wave signal and a radiation intensity will be simply described here.

Because the signal intensity of a photoacoustic wave is proportioned to the light quantity given to the living body tissue 101 as an object of the detection, it is preferable to illuminate the surface of the living body tissue 101 with a light of a large light quantity.

On the other hand, the light quantity capable of irradiating the surface of a living body is regulated as the maximum permissible exposure (MPE) by Japanese Industrial Standard (JIS). The MPE means the maximum permissible value of the light intensity (or light energy) irradiated per unit area.

It is required to radiate a light uniformly and efficiently onto the surface of a living body within the range not exceeding the MPE.

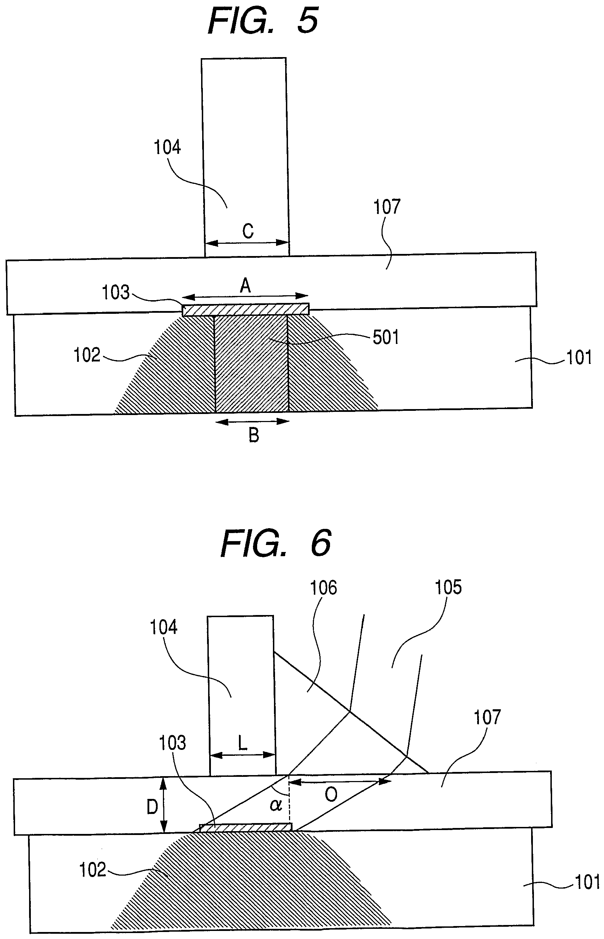

FIG. 5 illustrates a sectional view of the acquisition range and illumination region of an acoustic wave.

In the back detection PAT, as illustrated in FIG. 5, it is preferable to perform uniform illumination on the whole surface of the living body tissue 101 situated on the back of the acoustic wave detector 104 without forming any un-illumination regions.

Moreover, it is preferable to form a size C of the acoustic wave detector 104 to be wider than an imaging region B in order to acquire an acoustic wave from the absorber as an object at a high SN ratio at the time of processing the electric signal converted from the acoustic wave to image the positional information and the like of the absorber situated in a living body.

That is, as illustrated in FIG. 5, the intensity of an acoustic wave signal can be increased and the uniformity of the acoustic wave signal can be improved by forming an illuminating area A including an image reconstructing region 501 to be wider than the size C of the acoustic wave detector 104, and by forming the size C of the acoustic wave detector 104 to be wider than the imaging region B.

The region in which acoustic wave detection can be performed is the one within about 45° onto the outer side from the normal line direction of each element included in the acoustic wave detector 104, that is, the detectable angle is about 45°.

Hence, in the case of performing imaging by adding all the acoustic wave signals capable of being captured and by performing suitable imaging processing without considering signal uniformity and the like, the imaging region B sometimes becomes wider than the size C of the acoustic wave detector 104.

However, in this case, it is required to set the illuminating area A to a wider range than the imaging region B.

As illustrated in FIG. 4, in the case of performing illumination from a side of the acoustic wave detector 104, a light is obliquely advanced to the flat surface plate 107.

Although a little volume diffusion exists in the flat surface plate 107, vast volume diffusion is unsuitable for improving the transmittance of light, and it is preferable to increase rectilinear propagation components mainly.

A sectional view of an illuminating area 103 formed on the surface of the living body tissue 101 by a light going almost straight in the flat surface plate 107 is illustrated in FIG. 6 here.

Hereupon a character $\alpha$ denotes the angle of the advancing light, a character L denotes the width of the acoustic wave detector 104, a character D denotes the thickness of the flat surface plate 107, and a character O denotes the horizontal width of the light on the incidence plane.

Although not illustrated in FIG. 6, the sectional shape of the acoustic wave detector 104 is designed to be a regular square or a rectangle, and length of the light in the depth direction (paper surface direction) on the incidence plane is designed to be longer than the length of the depth (paper surface direction) length of the acoustic wave detector 104.

In order to form the desired light as illustrated in FIG. 5, it is required to satisfy the relation of: $D \times \tan(\alpha) \geq L$.

In the case where the width of the acoustic wave detector 104 is wide, it is required to set the angle $\alpha$ of the advancing light to be large and set the thickness D of the flat surface plate 107 to be thick.

On the other hand, because the acoustic wave is a spherical wave, the signal intensity of the acoustic wave is inversely proportional to the interval of an absorber and an acoustic wave detection device.

The acoustic wave is attenuated by a certain degree also in the flat surface plate 107. Hence, it is preferable to thin the thickness of the flat surface plate 107 in order to acquire a large acoustic wave signal. That is, it is preferable to form a necessary approach angle (angle $\alpha$ of the advancing light).

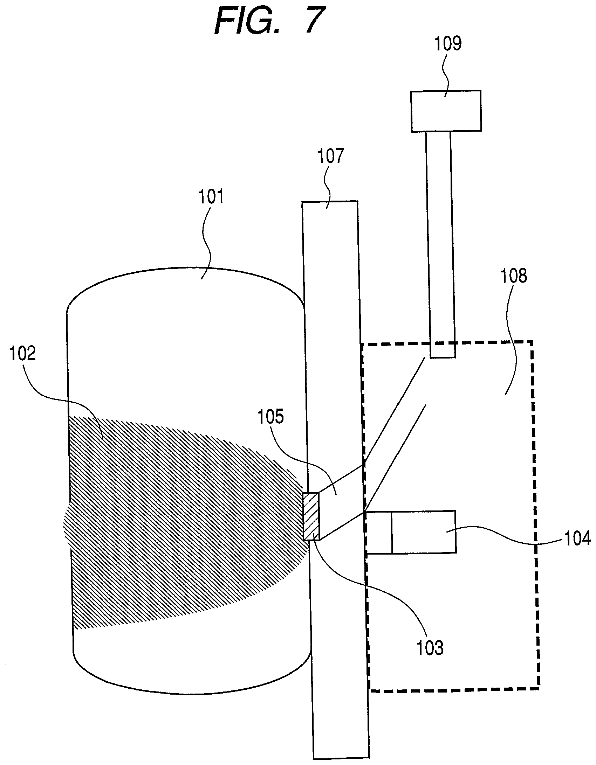

FIG. 7 illustrates a schematic view of an apparatus in which the light transmitting member 106 of the back detection PAT of FIG. 4 is removed.

As illustrated in FIG. 7, in the case of entering a light from the atmosphere to the flat surface plate 107 directly, the refracting angle of the light to the flat surface plate 107 becomes the advancing angle of the light in the flat surface plate 107 here. The approach angle $\beta1$ and refracting angle $\beta2$ of the light follow Snell's law: $n1 \times \sin(\beta1) = n2 \times \text{sine}(\beta2)$, where a character n1 denotes the refractive index of the atmosphere, and a character n2 denotes the refractive index of the flat surface plate 107. The material of the flat surface plate 107 is required to be able to cope with both the transparency of an acoustic wave and light.

For the aspect of light transparency, resin materials, such as acrylic, polycarbonate, and polymethylpentene, and glass materials are given. As the material, glass (BK7) has almost 100% of transmittance in near-infrared and visible regions. However, reflection arises on the surface of glass, and consequently four to five percent of reflection is generally caused on both the front surface and back surface of the glass having no antireflective film coat. On the other hand, by coating an antireflective film on the front surface in order to increase the transmittance, 99% or more of transmittance can be secured. In the case of using a resin material, the transmittance of the material itself is considerably high to be near to 100% (in near-infrared and visible regions). However, also in this case, there are reflections of about four percent both on the front and back surfaces owing to the surface reflections according to the material, and accordingly it is preferable to coat an antireflective film. Moreover, if also the acoustic wave characteristic is considered, polymethylpentene is a more preferable material.

Although the case where the refractive index of polymethylpentene is 1.463 is exemplified here, all of the refractive indices of the above mentioned materials are in the neighborhood of 1.5 (there are extensive options of refractive indices of optical glass materials).

If a light enters from a medium having a small refractive index into a medium having a large refractive index, the refracting angle at that time cannot be made to be large, and the refracting angle of a light entering polymethylpentene from the atmosphere is up to 40° at a maximum. That is, the advancing angle of 40° or more cannot be acquired in the configuration illustrated in FIG. 7.

On the other hand, by making a light from the light source enter the inclined plane of the light transmitting member 106, as illustrated in FIG. 4, in place of making the light enter the flat surface plate 107 parallel to the surface of the living body tissue 101, the limitation of the advancing angle of the light in the flat surface plate 107 as illustrated in FIG. 7 is removed.

That is, in case of using a prism or the like made of a material having high light transparency, such as optical glass, as the light transmitting member 106, the refractive index of the material can be selected in a wide range from the neighborhood of 1.5 to 2 or more.

As described above, if the refractive index of the material constituting the flat surface plate 107 is made to be in the neighborhood of 1.5, then the refractive indices of the light transmitting member 106 and the flat surface plate 107 become equal to each other, or the refractive index of the light transmitting member 106 becomes larger than that of the flat surface plate 107 here.

Hence, the advancing angle of the light in the flat surface plate 107 can be made to be large by Snell's law.

Since the refractive index of the light transmitting member 106, which is the incidence plane of the light from the atmosphere, is large, the refracting angle thereof becomes small. However, the inclination angle of the incidence plane can be arbitrarily determined, and also the incident angle of light can be arbitrarily determined.

Consequently, it is possible to set the advancing angle of light necessary in the flat surface plate 107 according to the width of the acoustic wave detector 104 and the thickness of the flat surface plate 107, and to form the refracting angle produced at the interface between the light transmitting member 106 and the flat surface plate 107 so as to be the advancing angle.

In the case of the present embodiment, it is necessary to incline the surface, from which the light enters the light transmitting member 106, to the flat surface plate 107. To incline indicates that the inclination angle is within a range of 0° ≤ inclination angle < 90° in the case where the normal line direction of the flat surface plate 107 is taken as a reference.

If a plane parallel to the flat surface plate 107 is produced and the light is made to enter the plane, the relation between the incident angle from the atmosphere and the refracting angle to the flat surface plate 107 is kept because the light does not enter by way of the light transmitting member as illustrated in FIG. 7.

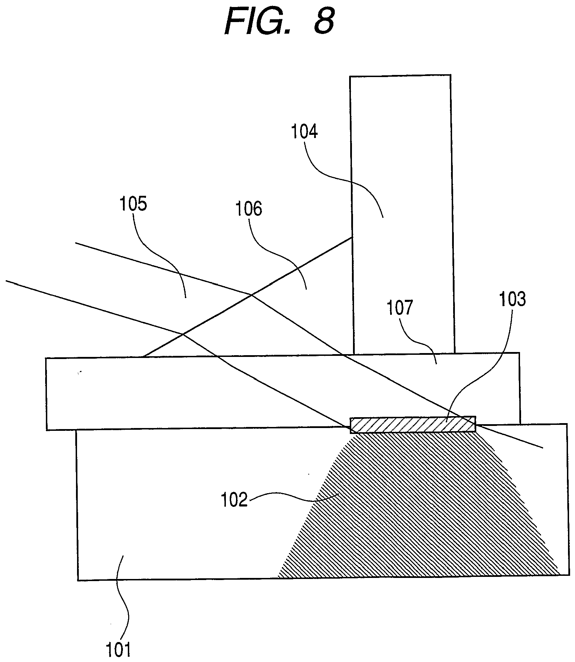

Consequently, the advancing angle of the light in the flat surface plate 107 cannot be made to be large. As an example, FIG. 8 illustrates a path of a light passing through each member.

FIG. 8 is an example to the last, and it is possible to make the light path the desired light path.

An outline of the radiation path of a light radiated from the light source to the living body tissue 101 is again given here.

A laser light generated by a pulse laser as the light source propagates in a space (the atmosphere) to enter the light transmitting member 106.

Because a refractive index difference is large on the incidence plane of the light transmitting member 106, the reflectance of light is large on the basis of Fresnel's Law.

It is necessary to produce a suitable antireflective film in order to make the light enter the light transmitting member 106 efficiently.

The light passing through the light transmitting member 106 enters the flat surface plate 107 without being shielded by the acoustic wave detector 104, but a thin lubricant is supplied between the flat surface plate 107 and the light transmitting member 106.

In order that the light may enter the flat surface plate 107, it is necessary to make the lubricant to have a suitable refractive index to the refractive indices of the light transmitting member 106 and the flat surface plate 107.

As to the lubricant the following points are necessary to be minded.

First, it is required to set the refractive index not to cause any total reflection when a light enters the flat surface plate 107 from the light transmitting member 106 through the lubricant.

Although the condition of the total reflection depends on an incident angle, the total reflection becomes easily caused even at a small incident angle as the refractive index greatly decreases from the interface to which the light enters as a boundary.

Second, it is required to reduce the reflectance produced at each interface to make the light enter efficiently. It is preferable that an antireflective film is provided to each interface, and the antireflective film is required to have durability because the unit structure 108 of the integration of the acoustic wave detector 104 and the light transmitting member 106 is operated to contact the flat surface plate 107 here.

Consequently, it is more preferable to decrease the reflectance without forming the antireflective film. Hence, it is a preferable form to decrease the reflectance by means of the lubricant because the reflectance decreases when the refractive index of the lubricant is made to be in the neighborhood of the refractive indices of the light transmitting member 106 and the flat surface plate 107.

The light enters the living body tissue 101 through the flat surface plate 107. The refractive index of the living body tissue 101 is not constant when the reflection of the skin of the living body tissue 101 is included into the refractive index, and consequently it is difficult to remove the reflection completely.

Because the living body tissue 101 includes much moisture, the refractive index of the living body tissue 101 is assumed to be the same as the refractive index of water, 1.33, here.

As illustrated in the example of FIG. 8, the material of the flat surface plate 107 is a resin material having a refractive index of being in the neighborhood of 1.5, and also the refractive index of the exemplified polymethylpentene is 1.463.

If the refractive index of the living body tissue 101 is assumed to be 1.33, then the light will enter the medium having a smaller refractive index. Hence, the advancing angle of light in the flat surface plate 107 must not be made too so that the total reflection would not occur.

Moreover, because the reflectance also significantly increases as the incident angle and the refracting angle become larger, it is preferable to form an antireflective film on this interface.

As described above, in order to improve the transmittance of light to radiate the light into the living body tissue 101 efficiently, it is desirable to design the structure and material under the consideration of the transmission, refraction, and reflection of the light caused by material characteristics.

In the above description, the example in which the light entering the inclined plane of the light transmitting member 106 arranged on the side of the acoustic wave detector 104 goes straight on in the inner part of the light transmitting member 106 to enter the flat surface plate 107 has been described.

However, the present invention is not limited to the case where the light goes straight on in the inner part of the light transmitting member 106.

For example, after a light has entered the light transmitting member 106, the light may be reflected on an inclined plane in the inner part of the member 106, that is, the light may be internally reflected, and may enter the flat surface plate 107.

In such a case, it is also allowed that the incidence plane of the light transmitting member 106 is parallel to the flat surface plate 107, and no angle limitation of the incidence plane of the light transmitting member 106 to the flat surface plate 107 arises.

In this case, it is a necessary condition that a light is internally reflected on at least one inclined plane other than the incidence plane in the light transmitting member 106.

(Second Embodiment)

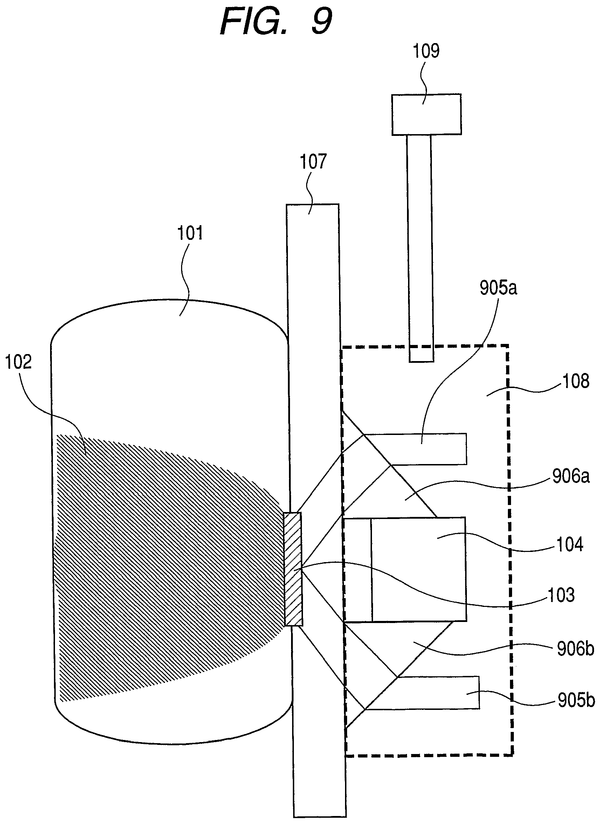

Next, as a second embodiment, a configuration example of the back detection PAT is described. FIG. 9 illustrates the configuration example of the back detection PAT according to the second embodiment.

The apparatus of the back detection PAT according to the present embodiment includes two light transmitting members 906a and 906b differently from the apparatus illustrated in FIG. 4.

Illuminating lights radiated from a light source to the present apparatus pass through illuminating light paths 905a and 905b, respectively.

The two light transmitting members 906a and 906b are fixed on both the sides of the acoustic wave detector 104 and are integrated to form the unit structure 108 here.

The unit structure 108 two-dimensionally scans the flat surface plate 107 through a lubricant, which is used also as an acoustic wave matching agent, and thereby detects an acoustic wave signal generated from an absorber in the living body tissue 101.

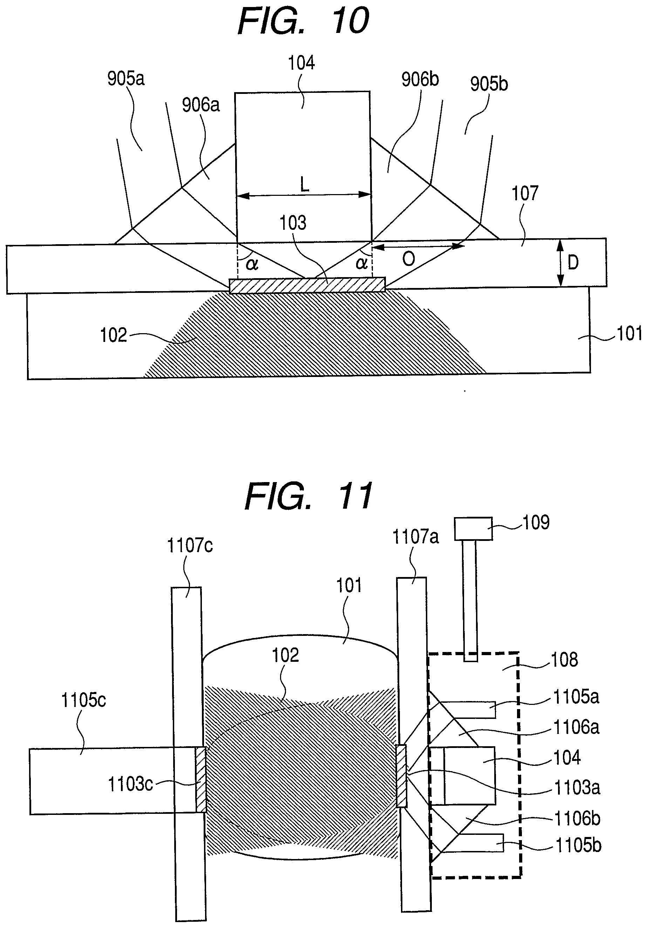

FIG. 10 illustrates the sectional view of the illuminating area 103 formed on the surface of the living body tissue 101.

FIG. 6 and FIG. 10 are different from each other in the numbers of the light transmitting members 106, and 906a and 906b.

Similarly to FIG. 6, the angle of the advance of a light is denoted by the character $\alpha$, the width of the acoustic wave detector 104 is denoted by the character L, the thickness of the flat surface plate 107 is denoted by the character D, and the horizontal width of the light on an incidence plane is denoted by a character O. The acoustic wave detector 104 is designed to be a regular square or a rectangle, and the length of the light in the depth direction (paper surface direction) on the incidence plane is set to be longer than the length of the acoustic wave detector 104 in the depth (paper surface direction).

In order to form a desired light as illustrated in FIG. 5, it is necessary that the horizontal width O of an incident light satisfies the relation of O > L/2, and the thickness D, the angle $\alpha$, and the width L must satisfy the relation of $D \times \tan(\alpha) \geq L/2$.

In the case of making lights enter the living body tissue 101 from both the sides of the acoustic wave detector 104, the lights entering the tissue 101 from both the sides enter up to a half of the width L of the acoustic wave detector 104, and the lights entering the tissue 101 from both the sides irradiate the whole surface of the surface of the living body tissue 101 situated on the back of the acoustic wave detector 104.

In comparison with the case illustrated in FIG. 6, the case of the present embodiment can make the advancing angle $\alpha$ smaller under the condition that the thickness D and the width L are respectively the same in both the cases.

As the advancing angle $\alpha$ becomes smaller, the attenuation of the transmittance of light in the flat surface plate 107 can be made to be less.

Moreover, the reductions of the incident angle and refracting angle at the interface between the flat surface plate 107 and the living body tissue 101 causes the reduction of reflectance, and the reductions are more preferable.

In the condition in which the width L and the angle $\alpha$ are the same, the thickness D of the flat surface plate 107 becomes thin, and brings about the improvement of the acoustic wave signal intensity as described with reference to FIG. 6.

(Third Embodiment)

Next, as a third embodiment, a configuration example of both-side irradiation PAT will be described. FIG. 11 illustrates the configuration example of the both-side irradiation PAT according to the third embodiment.

The both-side irradiation PAT of the present embodiment takes the form of the combination of the back detection PAT of the second embodiment and forward detection PAT of making a light enter the tissue 101 from the opposite side of the detector 104.

In the present embodiment, an apparatus includes an illuminating area 1103a from the side of the acoustic wave detector 104 and an illuminating area 1103c from the opposed side of the acoustic wave detector 104.

Furthermore, the apparatus includes a flat surface plate 1107a, which is an object holding member on the side of the acoustic wave detector 104, and a parallel flat plate 1107c on the opposed side of the acoustic wave detector 104, the acoustic wave detector 104, two light transmitting members 1106a and 1106b, and light sources.

The light sources illuminate the tissue 101 with lights through illuminating light paths 1105a, 1105b, and 1105c in the present apparatus. As the light sources, different pulse lasers may be prepared on the side of the acoustic wave detector 104 and the opposed side thereof, or the same pulse laser may be prepared.

In the case of using two different pulse lasers, a jitter, which is a difference between radiation times, depends on the sizes of detection objects, and only the thing required for the pulse lasers is to radiate pulse laser lights with the jitters less than 100 nanoseconds in order to acquire several mm of resolution.

The technique of putting a breast between the flat surface plates 1107a and 1107c to press the breast with the flat surface plates 1107a and 1107c and of diagnosing the breast in the state of being thinned is an effective technique capable of compensating the limitation of the resolution of the depth of the PAT. However, in the case of the illumination of one surface side, the breast in the neighborhood of the acoustic wave detector 104 corresponds to a deep part to the front surface on which a light is radiated.

Hence, the light quantity reaching an absorber (neovascular vessels here) situated in the breast in the neighborhood of the acoustic wave detector 104 remarkably decreases, and signal intensity becomes small.

On the other hand, in the case of the both-side irradiation PAT, if the same light quantities are illuminated from both surfaces, the minimum signal part is the living body part at the midsection of the flat surface plates 1107a and 1107c.

That is, if the forward detection PAT and the both-side irradiation PAT are compared with each other under the condition that the using light quantities are the same and the thicknesses of the living body tissues 101 are constant, then the both-side irradiation PAT is obviously advantageous on the light availability.

The third embodiment is an embodiment capable of realizing a method of efficiently measuring the whole living body tissue 101 by adopting the both-side irradiation PAT, adjusting the thickness of the living body tissue 101 to be measured with two flat surface plates 1107a and 1107c.

According to the configuration of the present embodiment described above, the lights illuminating the tissue 101 from the same side of the acoustic wave detector 104 form the illuminating areas on the whole surface of the back side of the acoustic wave detector 104.

Furthermore, by performing light irradiation from both the surfaces of the living body tissue 101 put between the flat surface plates 1107a and 1107c, the efficient illumination to the whole living body tissue 101 is enabled, and accurate measurement of a photoacoustic signal is enabled.

The first to third embodiments described above have given outlines of the case where the light transmitting member 106 or the light transmitting members 906a and 906b or 1106a and 1106b are situated on the side or the sides of the 2-dimensional array acoustic wave detecting device 104 arranging elements having the shape of a regular square or a rectangle.

However, the configuration of dividing the 2-dimensional array acoustic wave detecting device 104 into two or more parts and putting the light transmitting member 106 between the 2-dimensional array acoustic wave detecting devices 104 may be adopted besides the above case.

That is, the biological information acquisition apparatus of the present invention efficiently radiates a light to the living body tissue 101 on the back of the elements of the acoustic wave detector 104 having various shapes by means of the light transmitting member 106 or the light transmitting members 906a and 906b or 1106a and 1106b, arranged on the side or sides of the acoustic wave detector 104 as an optical waveguide.

<Examples>

In the following, examples of the present invention will be described.

<Example 1>

In an example 1, a configuration example of back detection PAT to which the present invention is applied will be described. To the living body tissue 101, the back detection PAT composed of the flat surface plate 107, the acoustic wave detector 104, and the light transmitting member 106, configured as illustrated in FIG. 4, was prepared.

As the light source, Nd: YAG pulse laser having the oscillation wavelength of 1064 nm was used.

The acoustic wave detector 104 and the light transmitting member 106 were formed as the integrated unit structure 108 here.

As the flat surface plate 107, polymethylpentene having the refractive index of 1.463 was used, and the thickness thereof was set to 12.5 mm.

An antireflective film was formed on the contact surface of the flat surface plate 107 with the living body tissue 101 on the supposition that the refractive index of the living body tissue 101 was 1.33.

As the acoustic wave detector 104, an array detector arranging acoustic wave detection elements in two dimensions was used, and the size of the detection surface was 23 mm in breadth by 46 mm in length.

The horizontal width L of the acoustic wave detector 104 illustrated in FIG. 4 was 23 mm. A triangular prism made of BK7, which was an optical glass material available from NEC SCHOTT Components Corporation, was used as the light transmitting member 106. The refractive index of the prism was 1.507.

The inclined plane of the triangular prism from which a light entered was set at the angle of about 61° to the normal line direction of the flat surface plate 107. On the inclined plane, an antireflective film fitted to the incident angle of a light was coated.

Because the incident angle was set to about 45° to the inclined plane, the antireflective film was made the one corresponding to the angle here.

A reflection film was coated on each of the surfaces except the bottom surface of the prism, from which a light was emitted, and the inclined plane.

An acoustic wave signal was acquired by the scanning of the unit structure 108 on the flat surface plate 107, and castor oil was supplied between the flat surface plate 107 and the unit structure 108.

The refractive index of the castor oil was about 1.48. The magnitude relation of the refractive indices was: prism > castor oil > polymethylpentene.

A laser pulse light entering the inclined plane of the prism at about 45° advanced in the prism at the refracting angle of about 27°, and entered the interface with the flat surface plate 107. The incident angle corresponded to about 56° on the basis of the interface.

Because the refractive indices of the prism, the castor oil, and the flat surface plate 107 were the values approximate to one another, the contribution of the reflection was low, and the light advanced in the flat surface plate 107 at the angle of about 61° to the normal line direction of the flat surface plate 107.

A phantom made of a diffusion medium, which imitated a living body tissue and was 20 mm long, 40 mm wide, and 20 mm deep, was prepared.

When an acoustic wave signal was detected from the phantom with the PAT apparatus of the present example, the whole back of the detection surface of the acoustic wave detector 104 could be illuminated, and a good acoustic signal could be acquired from an absorber set in the phantom.

<Example 2>

In an example 2, a configuration example of back detection PAT of a form different from the example 1, to which the present invention was applied, will be described.

To the living body tissue 101, the back detection PAT composed of the flat surface plate 107, the acoustic wave detector 104, the light transmitting members 906a and 906b, configured as illustrated in FIG. 9, was prepared.

Lights were illuminated to the living body tissue 101 from both the sides of the acoustic wave detector 104 here. As the light source, Nd: YAG pulse laser having the oscillation wavelength of 1064 nm was used.

The two light transmitting members 906a and 906b were fixed on both the sides of the acoustic wave detector 104, and the acoustic wave detector 104 and the light transmitting members 906a and 906b were formed as the integrated unit structure 108 here.

The flat surface plate 107 was the same as that in the example 1. An array detector having acoustic wave detection elements arranged in two dimensions was used as the acoustic wave detector 104, and the size of the detection surface of the array detector was 44 mm wide and 44 mm long.

The same optical glass material as that of the example 1 was used as the light transmitting members 906a and 906b.

The same castor oil as that of the example 1 was supplied between the flat surface plate 107 and the unit structure 108. The advancing path of one of the light was the same as that of the example 1, while the advancing path of the other light was symmetrical with respect to the acoustic wave detector 104.

A phantom made of a diffusion medium, which imitated the living body tissue 101 and was 40 mm long, 40 mm wide, and 20 mm deep, was prepared. When an acoustic wave signal was detected from the phantom with the PAT apparatus of the present example, a good acoustic signal could be acquired from an absorber set in the phantom.

On the surface of the living body tissue 101, the laser lights irradiating the surface from both the sides illuminated the whole back of the detection surface of the acoustic wave detector 104, and consequently it was possible to illuminate the whole living body tissue 101.

In comparison with the example 1, the example 2 could use the acoustic wave detector 104 having a detection surface of a wider area, and consequently the area capable of being diagnosed at one time was widened to contribute the shortening of a diagnosis time.

<Example 3>

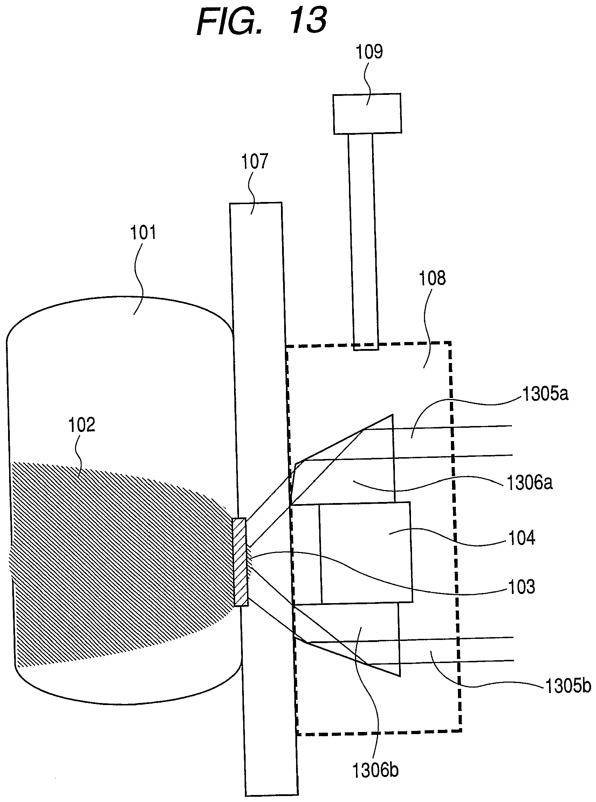

As illustrated in FIG. 13, the present example had the same configuration as that of the example 2 illustrated in FIG. 9, in which the unit structure 108 was formed by integrating the acoustic wave detector and the light transmitting members, except for the respect in which light transmitting members 1306a and 1306b substituted for the light transmitting members 906a and 906b.

The trapezoid-shaped light transmitting members 1306a and 1306b were used in the example 3 of FIG. 13 in place of the triangular light transmitting members 906a and 906b used in the example 2.

After laser lights had entered the light transmitting members 1306a and 1306b, the laser lights internally reflected on the flat inclined planes of the light transmitting members 1306a and 1306b and entered the flat surface plate 107 (through illuminating light paths 1305a and 1305b, respectively) here.

By setting the inclined planes to have desired angles to the flat surface plate 107, the incident angles of the laser lights entering the flat surface plate 107 could be adjusted to the same angles as those of the example 2, and consequently the example 3 had the advantages equal to those of the example 2.

<Example 4>

In an example 4, a configuration example of both-side irradiation PAT to which the present invention is applied will be described. The present example configured the both-side irradiation PAT as illustrated in FIG. 11.

The apparatus and the entering of lights on the acoustic wave detector 104 side were the same as those of the example 2 of FIG. 9, respectively.

The parallel flat plate 1107c was prepared on the side opposite to the acoustic wave detector 104, and the present example was configured to put the living body tissue 101 between the two flat plates 1107a and 1107c. A mechanism for controlling the pressed thickness of the living body tissue 101 was provided here.

A laser pulse light having an area wider than that of the detection surface of the acoustic wave detector 104 was radiated from the normal line direction of the parallel flat plate 1107c on the opposite side. The illumination axis of the laser pulse light was made to accord with the central axis of the acoustic wave detector 104. A phantom made of a diffusion medium which imitated the living body tissue 101 and was 40 mm long, 40 mm wide, and 40 mm deep was prepared, and an acoustic wave signal was detected from the phantom with the PAT apparatus of the present example. Thereby, a good acoustic wave signal could be acquired from an absorber installed in the phantom.

By adopting the configuration of the present example, more efficient illumination could be performed in comparison with the illumination only from one side surface.

In particular, by the both-side irradiation PAT, a strong acoustic wave signal could be acquired also from the living body tissue 101 in a deep part, which could not be efficiently illuminated by one-side irradiation.

(Comparative Example)

In the following, a comparative example to the present invention will be described.

The back detection PAT having the configuration illustrated in FIG. 7 was prepared to the living body tissue 101, which configuration included the flat surface plate 107 and the acoustic wave detector 104.

As the light source, a pulse laser made of Nd: YAG having an oscillation wavelength of 1064 nm was used.

As the material of the flat surface plate 107, polymethylpentene having a refractive index of 1.463 was used, and the thickness of the flat surface plate 107 was made 12.5 mm.

An antireflective film based on the supposition of the refractive index of the living body tissue 101 to be 1.33 was formed on the contact surface of the flat surface plate 107 with the living body tissue 101.

As the acoustic wave detector 104, an array detector arranging acoustic wave detection elements in two dimensions were used, and the size of the detection surface of the acoustic wave detector 104 was designed to be 23 mm wide and 46 mm long.

A laser light directly entered the flat surface plate 107 from the atmosphere, but an antireflective film corresponding to the incident angle of 80° was added to the flat surface plate 107. In the present comparative example, acoustic waves were measured without performing the scanning by the unit structure 108, and castor oil was supplied only between the acoustic wave detector 104 and the flat surface plate 107 in order to secure the transmission of acoustic waves. The refractive index of the castor oil was about 1.48.

A laser light entering the flat surface plate 107 at the incident angle of 80° had the refracting angle of 40°, and advanced in the flat surface plate 107 at the angle of 40°.

A phantom made of a diffusion medium which imitated the living body tissue 101 and was 20 mm long, 40 mm wide, and 20 mm deep was prepared.

Because the whole back of the detection surface of the acoustic wave detector 104 could not be illuminated under the conditions described above, the signal from a region distant from the irradiated surface in the absorber installed in the phantom particularly deteriorated. Consequently, it was difficult to acquire good acoustic wave signals by the comparative example in comparison with the example 1 as described above.

<Example 5>

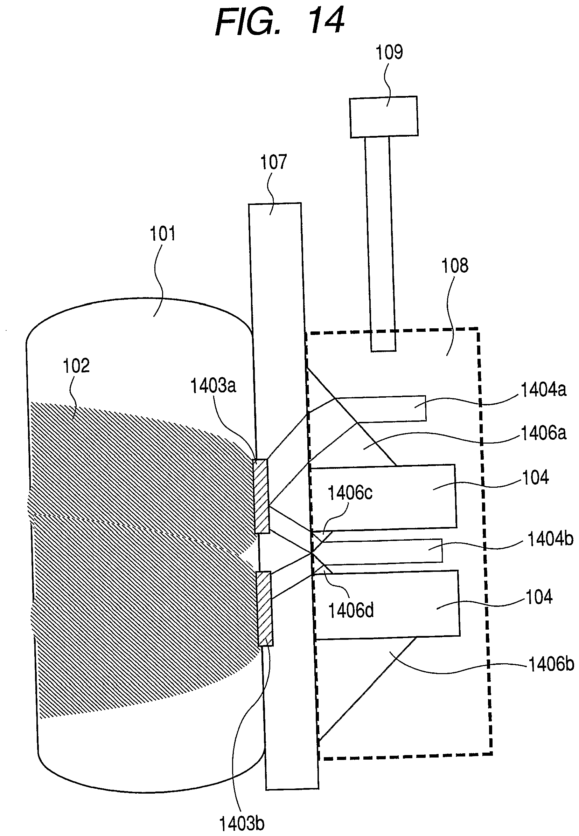

As illustrated in FIG. 14, in the present example, the acoustic wave detector 104 was divided into two parts corresponding to illuminating areas 1403a and 1403b.

Light transmitting members 1406a, 1406b, 1406c, and 1406d were provided on both sides of the outer side of the acoustic wave detectors 104, and between the acoustic wave detectors 104, respectively, and these acoustic wave detectors 104 and the light transmitting members 1406a, 1406b, 1406c, and 1406d were integrated to form a unit structure 108.

Similarly to the case illustrated in the example 2, the backs of the acoustic wave detectors 104 were illuminated from the outer side of the acoustic wave detectors 104 (e.g., illuminating light path 1404a), and also the light passing between the acoustic wave detectors 104 entered the inclined plane of the light transmitting members 1406c and 1406d. After that, the light entered the living body tissue 101 with angles to illuminate the living body tissue 101 situated on the backs of the acoustic wave detectors 104 (illuminating light path 1404b).

Consequently, similarly to the example 2, the whole living body tissue 101 could be efficiently illuminated.

**Claims**

1. An information acquisition apparatus for detecting an acoustic wave generated from an object by making a light from a light source enter the object, the information acquisition apparatus comprising:

the light source;
an acoustic wave detector (104) including acoustic wave detection elements arranged in a two-dimensional array form, the acoustic wave detector having a width L in a first direction;
a prism (106) arranged alongside the acoustic wave detector (104) in the first direction so as to form an irradiation area (103) based on the light from the light source at a position of the object opposed to the acoustic wave detection elements; and

a flat surface plate (107) for allowing the light emitted from the prism (106) to pass through the flat surface plate (107) to thereby guide the light to the irradiation area (103) of the object, the flat surface plate (107) having a thickness D, wherein the light from the light source enters the prism (106) from a side surface of the acoustic wave detector (104) and enters the flat surface plate (107) through the prism at an angle $\alpha$ other than one perpendicular to the flat surface plate, and

wherein $D \times \tan(\alpha) \geq L$ is satisfied so that the size of the irradiation area (103) in the first direction is larger than the width L of the acoustic wave detector (104).

2. The information acquisition apparatus according to claim 1, wherein the prism (106) is so configured that the irradiation area (103) includes a whole region on a surface of the object corresponding to a back of a detection surface of the acoustic wave detector (104).

3. The information acquisition apparatus according to claim 1 or 2, wherein the flat surface plate (107) is arranged between the light source and the object.

4. The information acquisition apparatus according to any one of claims 1 to 3, wherein the acoustic wave detector (104) and the prism (106) are integrated to form a unit structure, and the unit structure is configured to be subjected to two-dimensional scanning.

5. The information acquisition apparatus according to claim 1, comprising:
the light source, wherein the light source is configured to generate a pulse light.

6. The information acquisition apparatus according to claim 5, wherein the prism is a triangular prism.

7. An information acquisition apparatus for detecting an acoustic wave generated from an object by making a light from a light source enter the object, the information acquisition apparatus comprising:

the light source;
an acoustic wave detector (104) including acoustic wave detection elements arranged in a two-dimensional array form, the acoustic wave detector having a width L in a first direction;
a first prism (906a) arranged alongside the acoustic wave detector (104) in the first direction so as to form a first irradiation area (103) based on the light from the light source at a position of the object opposed to the acoustic wave detection elements;

a second prism (906b) arranged on a side opposite to the first prism (906a) with the acoustic wave detector (104) between the first and second prisms (906a, 906b) so as to form a second irradiation area (103) based on the light from the light source at a position of the object opposed to the acoustic wave detection elements; and

a flat surface plate (107) for allowing the light emitted from the first prism (906a) to pass through the flat surface plate (107) to thereby guide the light to the first irradiation area (103) of the object and allowing the light emitted from the second prism (906b) to pass through the flat surface plate (107) to thereby guide the light to the second irradiation area (103) of the object, the flat surface plate (107) having a thickness D, wherein the light from the light source enters the first prism (906a) from a side surface of the acoustic wave detector (104) and enters the flat surface plate (107) through the first prism at an angle $\alpha$ other than one perpendicular to the flat surface plate, and wherein the light from the light source enters the second prism (906b) from a side surface of the acoustic wave detector (104) and enters the flat surface plate (107) through the second prism at an angle $\alpha$ other than one perpendicular to the flat surface plate, and

wherein $D \times \tan(\alpha) \geq L/2$ is satisfied so that the size of the total region of the first and second irradiation areas (103) in the first direction is larger than the width L of the acoustic wave detector (104).

**Patentansprüche**

1. Informationsbeschaffungsvorrichtung zur Erfassung einer akustischen Welle, die von einem Objekt erzeugt wird, indem Licht von einer Lichtquelle zum Eintreten in das Objekt veranlasst wird, wobei die Informationsbeschaffungsvorrichtung umfasst:

die Lichtquelle,
eine Akustikwellenerfassungseinrichtung (104) mit Akustikwellenerfassungselementen, die in einer zweidimensionalen Array-Form angeordnet sind, wobei die Akustikwellenerfassungseinrichtung eine Breite L in einer ersten Richtung aufweist,
ein Prisma (106), das längs der Akustikwellenerfassungseinrichtung (104) in der ersten Richtung zur Ausbildung eines Bestrahlungsbereichs (103) beruhend auf dem Licht von der Lichtquelle an einer Position des Objekts angeordnet ist, die den Akustikwellenerfassungselementen gegenüberliegt, und

eine Flache-Oberfläche-Platte (107), um dem vom Prisma (106) emittierten Licht ein Durchlaufen der Flache-Oberfläche-Platte (107) zu erlauben, um das Licht so zu dem Bestrahlungsbereich (103) des Objekts zu führen, wobei die Flache-Oberfläche-Platte (107) eine Dicke D aufweist,

wobei das Licht von der Lichtquelle von einer Seitenfläche der Akustikwellenerfassungseinrichtung (104) in das Prisma (106) eintritt und über das Prisma bei einem Winkel $\alpha$ in die Flache-Oberfläche-Platte (107) eintritt, der von einem rechten Winkel zu der Flache-Oberfläche-Platte verschieden ist, und

wobei $D \times \tan(\alpha) \geq L$ erfüllt ist, sodass die Größe des Bestrahlungsbereichs (103) in der ersten Richtung größer als die Breite L der Akustikwellenerfassungseinrichtung (104) ist.

2. Informationsbeschaffungsvorrichtung nach Anspruch 1, wobei das Prisma (106) derart konfiguriert ist, dass der Bestrahlungsbereich (103) eine gesamte Region einer Oberfläche des Objekts enthält, die einer Hinterseite einer Erfassungsfläche der Akustikwellenerfassungseinrichtung (104) entspricht.

3. Informationsbeschaffungsvorrichtung nach Anspruch 1 oder 2, wobei die Flache-Oberfläche-Platte (107) zwischen der Lichtquelle und dem Objekt angeordnet ist.

4. Informationsbeschaffungsvorrichtung nach einem der Ansprüche 1 bis 3, wobei die Akustikwellenerfassungseinrichtung (104) und das Prisma (106) zum Bilden eines Einheitsaufbaus integriert sind, und der Einheitsaufbau konfiguriert ist, einer zweidimensionalen Abtastung unterzogen zu werden.

5. Informationsbeschaffungsvorrichtung nach Anspruch 1, mit
der Lichtquelle, wobei die Lichtquelle zur Erzeugung von Pulslicht eingerichtet ist.

6. Informationsbeschaffungsvorrichtung nach Anspruch 5, wobei das Prisma ein Dreiecksprisma ist.

7. Informationsbeschaffungsvorrichtung zur Erfassung einer akustischen Welle, die von einem Objekt erzeugt wird, indem Licht von einer Lichtquelle zum Eintreten in das Objekt veranlasst wird, wobei die Informationsbeschaffungsvorrichtung umfasst:

die Lichtquelle,
eine Akustikwellenerfassungseinrichtung (104) mit Akustikwellenerfassungselementen, die in einer zweidimensionalen Array-Form angeordnet sind, wobei die Akustikwellenerfassungseinrichtung eine Breite L in einer ersten Richtung aufweist,

ein erstes Prisma (906a), das längs der Akustikwellenerfassungseinrichtung (104) in der ersten Richtung angeordnet ist, um einen ersten Bestrahlungsbereich (103) beruhend auf dem Licht von der Lichtquelle an einer Position des Objekts zu bilden, die den Akustikwellenerfassungselementen gegenüberliegt,

ein zweites Prisma (906b), das auf einer Seite dem ersten Prisma (906a) gegenüberliegend angeordnet ist, wobei sich die Akustikwellenerfassungseinrichtung (104) zwischen dem ersten Prisma (906a) und dem zweiten Prisma (906b) befindet, um einen zweiten Bestrahlungsbereich (103) beruhend auf dem Licht von der Lichtquelle an einer Position des Objekts zu bilden, die den Akustikwellenerfassungselementen gegenüberliegt, und

eine Flache-Oberfläche-Platte (107), um dem vom ersten Prisma (906a) emittierten Licht ein Durchlaufen der Flache-Oberfläche-Platte (107) zu ermöglichen, um das Licht so zu dem ersten Bestrahlungsbereich (103) des Objekts zu führen, und um dem vom zweiten Prisma (906b) emittierten Licht ein Durchlaufen der Flache-Oberfläche-Platte (107) zu ermöglichen, um das Licht so zu dem zweiten Bestrahlungsbereich (103) des Objekts zu führen, wobei die Flache-Oberfläche-Platte (107) eine Dicke D aufweist,

wobei das Licht von der Lichtquelle von einer Seitenfläche der Akustikwellenerfassungseinrichtung (104) in das erste Prisma (906a) eintritt und über das erste Prisma bei einem Winkel $\alpha$ in die Flache-Oberfläche-Platte (107) eintritt, der von einem senkrechten Winkel zur Flache-Oberfläche-Platte verschieden ist, und wobei das Licht von der Lichtquelle von einer Seitenfläche der Akustikwellenerfassungseinrichtung (104) in das zweite Prisma (906b) eintritt und über das zweite Prisma bei einem Winkel $\alpha$ in die Flache-Oberfläche-Platte (107) eintritt, der von einem rechten Winkel zu der Flache-Oberfläche-Platte verschieden ist, und

wobei $D \times \tan(\alpha) \geq L/2$ erfüllt ist, sodass die Größe der Gesamtregion des ersten und des zweiten Bestrahlungsbereichs (103) in der ersten Richtung größer als die Breite L der Akustikwellenerfassungseinrichtung (104) ist.

**Revendications**

1. Appareil d'acquisition d'informations destiné à détecter une onde acoustique générée à partir d'un objet en amenant une lumière issue d'une source de lumière à pénétrer l'objet, l'appareil d'acquisition d'informations comprenant :

la source de lumière ;

un détecteur d'onde acoustique (104) comprenant des éléments de détection d'onde acoustique disposés sous une forme de réseau bidimensionnel, le détecteur d'onde acoustique ayant une largeur L dans une première direction ;

un prisme (106) disposé le long du détecteur d'onde acoustique (104) dans la première direction de façon à former une zone d'exposition à un rayonnement (103) sur la base de la lumière issue de la source de lumière au niveau d'une position de l'objet opposée aux éléments de détection d'onde acoustique ; et

une plaque à surface plate (107) destinée à permettre à la lumière émise à partir du prisme (106) de traverser la plaque à surface plate (107) pour ainsi guider la lumière vers la zone d'exposition à un rayonnement (103) de l'objet, la plaque à surface plate (107) ayant une épaisseur D,

dans lequel la lumière issue de la source de lumière pénètre le prisme (106) depuis une surface latérale du détecteur d'onde acoustique (104) et pénètre la plaque à surface plate (107) en traversant le prisme à un angle $\alpha$ autre qu'un angle perpendiculaire à la plaque à surface plate, et

dans lequel l'expression D x tan($\alpha$) $\geq$ L est satisfaite de sorte que la taille de la zone d'exposition à un rayonnement (103) dans la première direction soit supérieure à la largeur L du détecteur d'onde acoustique (104).

2. Appareil d'acquisition d'informations selon la revendication 1, dans lequel le prisme (106) est configuré de sorte que la zone d'exposition à un rayonnement (103) comprenne une région complète d'une surface de l'objet correspondant à l'arrière d'une surface de détection du détecteur d'onde acoustique (104).

3. Appareil d'acquisition d'informations selon la revendication 1 ou 2, dans lequel la plaque à surface plate (107) est disposée entre la source de lumière et l'objet.

4. Appareil d'acquisition d'informations selon l'une quelconque des revendications 1 à 3, dans lequel le détecteur d'onde acoustique (104) et le prisme (106) sont intégrés de façon à former une structure unitaire, et la structure unitaire est configurée pour être soumise à un balayage bidimensionnel.

5. Appareil d'acquisition d'informations selon la revendication 1, comprenant :
la source de lumière, dans lequel la source de lumière est configurée pour générer une lumière puisée.

6. Appareil d'acquisition d'informations selon la revendication 5, dans lequel le prisme est un prisme triangulaire.

7. Appareil d'acquisition d'informations destiné à détecter une onde acoustique générée à partir d'un objet en amenant une lumière issue d'une source de lumière à pénétrer l'objet, l'appareil d'acquisition d'informations comprenant :

la source de lumière ;

un détecteur d'onde acoustique (104) comprenant des éléments de détection d'onde acoustique disposés sous une forme de réseau bidimensionnel, le détecteur d'onde acoustique ayant une largeur L dans une première direction ;

un premier prisme (906a) disposé le long du détecteur d'onde acoustique (104) dans la première direction de façon à former une première zone d'exposition à un rayonnement (103) sur la base de la lumière issue de la source de lumière au niveau d'une position de l'objet opposée aux éléments de détection d'onde acoustique ;

un second prisme (906b) disposé sur un côté opposé du premier prisme (906a), le détecteur d'onde acoustique (104) se trouvant entre les premier et second prismes (906a, 906b) de façon à former une seconde zone d'exposition à un rayonnement (103) sur la base de la lumière issue de la source de lumière au niveau d'une position de l'objet opposée aux éléments de détection d'onde acoustique ; et

une plaque à surface plate (107) destinée à permettre à la lumière émise à partir du premier prisme (906a) de traverser la plaque à surface plate (107) pour ainsi guider la lumière vers la première zone d'exposition à un rayonnement (103) de l'objet et à permettre à la lumière émise à partir du second prisme (906b) de traverser la plaque à surface plate (107) pour ainsi guider la lumière vers la seconde zone d'exposition à un rayonnement (103) de l'objet, la plaque à surface plate (107) ayant une épaisseur D,

dans lequel, la lumière issue de la source de lumière pénètre le premier prisme (906a) depuis une surface latérale du détecteur d'onde acoustique (104) et pénètre la plaque à surface plate (107) en traversant le premier prisme à un angle $\alpha$ autre qu'un angle perpendiculaire à la plaque à surface plate, et dans lequel la lumière issue de la source de lumière pénètre le second prisme (906b) depuis une surface latérale du détecteur d'onde acoustique (104) et pénètre la plaque à surface plate (107) en traversant le second prisme à un angle $\alpha$ autre qu'un angle perpendiculaire à la plaque à surface plate, et dans lequel l'expression D x tan ($\alpha$) $\geq$ L/2 est satisfaite de sorte que la taille de la région totale des première et seconde zones d'exposition à un rayonnement (103) dans la première direction soit supérieure à la largeur L du détecteur d'onde acoustique (104).

## FIG. 1

## FIG. 2

# FIG. 3

# FIG. 4

## FIG. 5

## FIG. 6

# FIG. 7

FIG. 8

# FIG. 9

## FIG. 10

## FIG. 11

# FIG. 12

# FIG. 13

## FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008049063 A
- US 20060184042 A
- US 2006184042 A1
- WO 2005068973 A
- US 2005090725 A1
- US 5348002 A

**Non-patent literature cited in the description**

- **M. XU, L. V. WAHNG.** Photoacoustic imaging biomedicine. *Review of scientific instruments,* 2006, vol. 77, 04111
- **J. T. NIEDERHAUSER ; M. JEAGER ; R. LEMOR ; P. WEBER ; M. FRENZ.** *IEEE Transactions on medical imaging,* 2005, vol. 24 (4), 436